# EUROPÄISCHE PATENTSCHRIFT

(11) **EP 1 684 665 B1**
(45) Veröffentlichungstag und Bekanntmachung des Hinweises auf die Patenterteilung: **28.04.2010**
(21) Anmeldenummer: 04789959.6
(22) Anmeldetag: 11.10.2004
(51) Int. Cl.: A61F 2/90

(54) **STENT**
STENT
STENT

(30) Priorität: 10.11.2003 DE 10352874
(43) Veröffentlichungstag der Anmeldung: 02.08.2006
(73) Patentinhaber: Qualimed Innovative Medizinprodukte GmbH, 21423 Winsen (DE)
(72) Erfinder: NISSL, Thomas, 21441 Garstedt (DE)
(74) Vertreter: Ksoll, Peter
(86) Internationale Anmeldenummer: PCT/DE2004/002253
(87) Internationale Veröffentlichungsnummer: WO 2005/046522

(56) Entgegenhaltungen:
- WO-A1-01/89417
- WO-A2-00/32273
- DE-U1- 29 716 476

## Beschreibung

Die Erfindung betrifft einen Stent zur Behandlung von Stenosen.

Stenosen sind angeborene oder erworbene Gefäßverschlüsse oder Verengungen von tubulären Körperröhren, wie beispielsweise Luftröhre, Bronchien, Speiseröhre, Gallengänge, Harnwege, Aorta und Coronare oder andere Körpergefäße. Ursache für Stenosen sind häufig Tumore, die auf die Körperröhren drücken. Die Stenosen können durch operative und nicht operative Maßnahmen geöffnet werden. Bei den nicht operativen Maßnahmen werden Stents durch Kathetertechniken in das Gefäß im Bereich der Stenose eingebracht. Die Stents fungieren dort als Gefäßprothesen zur Abstützung der Gefäßinnenwände.

Stents gibt es in verschiedenartigsten Ausführungsformen und Designs des Stützgerüsts. Die WO 96/26689, die DE 297 02 671 U1 oder die DE 295 21 206 U1 werden als exemplarische Beispiele genannt. Ein Stent, der die Merkmale des Oberbegriffs des Anspruchs 1 aufweist, ist aus der WO 00/32273 bekannt.

Die Stents weisen ein tubuläres Stützgerüst aus Metall auf, welches aus mehreren Ringsegmenten besteht. Diese sind aus wellen- bzw. mäanderförmig sich über Bogenabschnitte endlos aneinander schließende Streben gebildet. In Längsachse des Stents benachbarte Ringsegmente sind durch Verbinder gekoppelt.

Zur Implantation werden die Stents zusammengedrückt. Fachsprachlich spricht man hierbei von Krimpen. Im gekrimpten Zustand werden die Stents mit Hilfe eines geeigneten Instruments in den Bereich der Stenose gebracht und dort abgesetzt, wobei das Stützgerüst vom Ausgangszustand in einen demgegenüber im Durchmesser vergrößerten Stützzustand aufweitbar ist. Dieser Aufweitvorgang kann selbsttätig bei sogenannten selbst expandierenden Stents erfolgen, er kann aber auch mit Hilfe eines geeigneten Werkzeugs, beispielsweise eines Ballonkatheters herbeigeführt werden.

In der Praxis hat sich herausgestellt, dass die Stützgerüste häufig entweder nicht die erforderlichen Radialkräfte bereitstellen, um einer Stenose ausreichend standzuhalten, oder aber so konfiguriert sind, dass sie die Funktion des Gefäßes behindern. Andere Ausführungsformen wiederum lassen sich nicht genügend krimpen oder sind im gekrimpten Zustand nicht flexibel genug, um problemlos den Körpergefäßen mit ihren Windungen folgen zu können.

Der Erfindung liegt daher ausgehend vom Stand der Technik die Aufgabe zugrunde, einen Stent mit möglichst hoher Stabilität, insbesondere Radialfestigkeit bei guter Ausnutzung des Materialeinsatzes zu schaffen.

Die Lösung dieser Aufgabe besteht nach der Erfindung in einem Stent gemäß den Merkmalen von Patentanspruch 1.

Der erfindungsgemäße Stent weist ein tubuläres Stützgerüst auf, welches von einem Ausgangszustand in einen Stützzustand aufweitbar ist. Das Stützgerüst besteht aus in Stentlängsachse aufeinander folgenden Ringsegmenten, die aus sich in Umfangsrichtung des Stützgerüstes wellenförmig endlos aneinanderschließenden Streben gebildet sind. Benachbarte Ringsegmente sind durch unterschiedlich lange Verbinder mit U-förmig konfigurierten Ausgleichsabschnitten gekoppelt. Diese Ausgleichsabschnitte weisen erfindungsgemäß alle in dieselbe Umfangsrichtung. Sowohl in Umfangsrichtung als auch in Stentlängsachse sind jeweils abwechselnd unterschiedlich lange Verbinder vorgesehen. Die Streben sind bogenförmig gekrümmt und gehen über Bogenabschnitte ineinander über, wobei alle Streben in dieselbe Umfangsrichtung gekrümmt sind. Dies ist vorteilhaft für den Krimpvorgang. Die langen Verbinder weisen beidseitig der Ausgleichsabschnitte bogenförmige Schenkel auf, die, dem Gesamtdesign entsprechend, in dieselbe Umfangsrichtung wie die Streben gekrümmt sind.

Dieses Design ist derart konfiguriert, dass im Stützzustand des Stents aus den in sich unverschieblichen Streben, die jeweils mit ihren Enden in den Knotenpunkten zusammenlaufen, wobei die Knotenpunkte als reibungsfreie Gelenke wirken, eine Art sich selbst stabilisierender Fachwerkverbund entsteht. Von außen bei Belastung des Stents wirkende Radialkräfte werden in den Knotenpunkten aufgenommen und dort in die verschiedenen Strebenrichtungen umgeleitet. Demzufolge zeichnet sich der erfindungsgemäße Stent durch eine hohe Stabilität und hohe Radialsteifigkeit aus. Dies kann genutzt werden, um die Materialdicke und die Strebenbreite des Stützgerüstes zu verringern, was nicht nur zu einer Verringerung des Materialeinsatzes führt, sondern insbesondere auch zu einer höheren Flexibilität und einer besseren Krimpbarkeit des Stents sowie einer besseren Restenoserate, wie bestehende Studien zeigen.

Der Stent kann aus Metall gefertigt sein. Hierbei können alle verformbaren medizinisch möglichen Metalle bzw. Metalllegierungen zum Einsatz gelangen, z.B. Edelstahl, Kobaltlegierungen (Phynox), Reineisen oder Nickel-Titan-Legierungen.

Besonders interessant ist der erfindungsgemäße Stent auch als Kunststoff-Stent, da diese im Allgemeinen keine große Festigkeit haben. Hier kommen insbesondere bioresorbierbare Kunststoffe zur Anwendung.

Die erfindungsgemäßen Stents werden für Körpergefäße unterschiedlichen Durchmessers jeweils abgestimmt im Design ausgelegt. Grundkonfiguration im Ausgangszustand sind die wellenförmig gestalteten Ringsegmente und die dazwischen eingegliederten Verbinder. Diese werden geometrisch, insbesondere hinsichtlich Länge der Schrägstreben und deren Winkelstellung zueinander, so ausgelegt, dass einerseits die Wellenberge und andererseits die Wellentäler benachbarter Ringsegmente einander frontal gegenüber liegen, wie dies Patentanspruch 2 vorsieht.

Die Verbinder sind in Stentlängsachse in Reihe hintereinander geschaltet, so dass sich ein wellenförmiges durchlaufendes Band aus Verbindern ergibt, wobei sich kurze und lange Verbinder alternierend abwechseln. Nach den Merkmalen von Patentanspruch 3 liegen hierbei die Anschlüsse der in Stentlängsachse aufeinander folgenden Verbinder an den Bogenabschnitten - also in den Knotenpunkten - einander frontal gegenüber. Auch diese Maßnahme wirkt sich positiv auf den Kraftverlauf im Stützgerüst aus, bei dem von außen angreifenden Kräfte an den Knotenpunkten aufgenommen und in die Streben umgeleitet werden sollen.

Eine sich theoretisch durch den Aufweitvorgang und den Übergang der Schrägstreben in eine gestreckte Form ergebende Längenverkürzung des Stützgerüstes wird durch die Ausgleichsabschnitte in den Verbindern ausgeglichen.

Insgesamt ergibt sich ein Stützgerüst mit hoher Radialsteifigkeit im Stützzustand. Dies gewährleistet eine sehr gute und homogene Schienung der Gefäßwand mit einer funktional zweckmäßigen Abstützung. Dennoch ist ein erfindungsgemäßer Stent sehr gut zu krimpen und weist in diesem Zustand eine glatte flexible Anordnung der Ringsegmente im Stützgerüst auf. Der Stent kann beispielsweise auf einem Ballonkatheter angeordnet sehr gut durch die Windungen eines Körpergefäßes gebracht werden. Diese Leichtgängigkeit bewirkt sowohl für den Anwender als auch für den Patienten eine hohe Sicherheit bei der Implantation.

Die Erfindung ist nachfolgend anhand eines in den Zeichnungen dargestellten Ausführungsbeispiels näher beschrieben. Es zeigen:
- Figur 1: das Stentmuster eines erfindungsgemäßen Stents im Ausgangszustand in einer Abwicklung;
- Figur 2: das Stentmuster im Stützzustand und
- Figur 3: das Stentmuster gemäß der Figur 2 mit der Darstellung des idealisiert angestrebten Fachwerkverbunds.

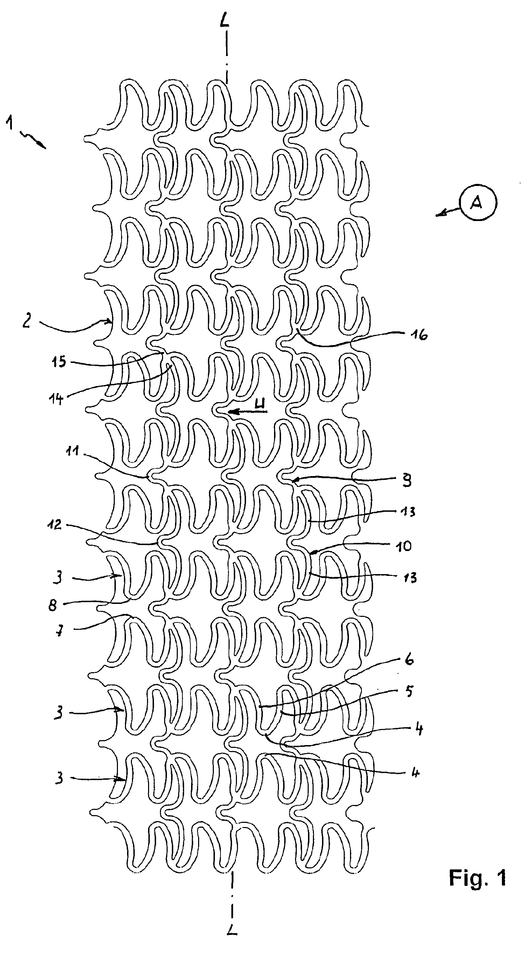

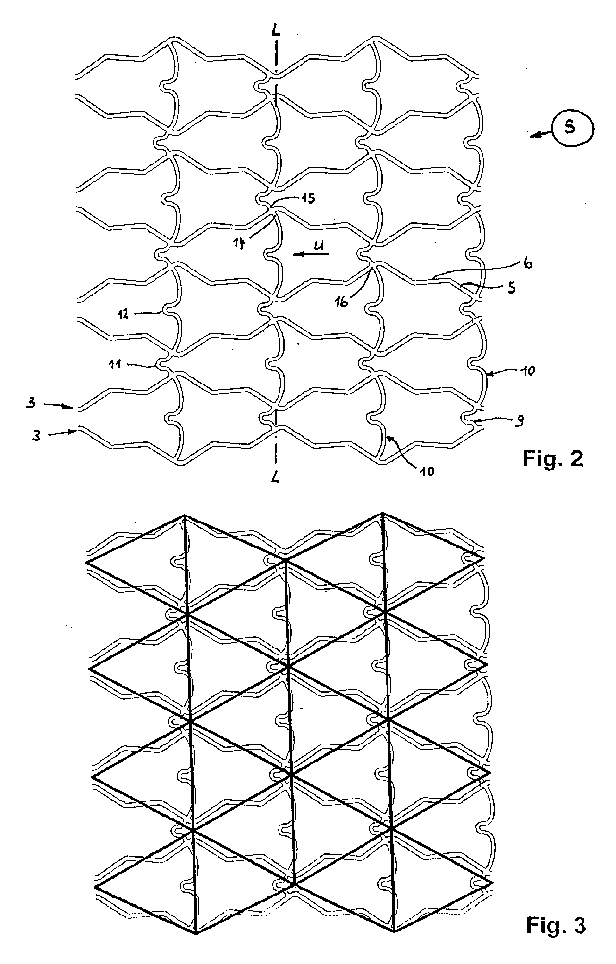

Die Figuren 1 und 2 zeigen das Stentmuster eines erfinderungsgemäß Stents 1 jeweils in einer Abwicklung. Figur 1 zeigt die Abwicklung des Stents 1 im Ausgangszustand A nach dessen Fertigung. Figur 2 gibt die Abwicklung des Stentmusters im aufgeweiteten Stützzustand S wieder.

Der Stent 1 ist aus Metall oder Kunststoff gefertigt und besitzt ein tubuläres Stützgerüst 2 aus mehreren hintereinander folgenden Ringsegmenten 3. Die Darstellungen der Figuren 1 und 2 sind nicht maßstäblich zu verstehen. Insbesondere zeigt Figur 2 nicht die gesamte Anzahl der Ringsegmente 3 des Stents 1 wie in Figur 1 dargestellt.

In seinem nicht expandierten Ausgangszustand A, wie in Figur 1 dargestellt, weisen die Ringsegmente 3 eine wellenförmige Konfiguration auf aus sich über Bogenabschnitte 4 endlos aneinander schließenden Streben 5, 6. Hierbei liegen einerseits die Wellenberge 7 und andererseits die Wellentäler 8 benachbarter Ringsegmente 3 einander frontal gegenüber. Untereinander sind die Ringsegmente 3 durch sich in Richtung der Stentlängsachse L erstreckende Verbinder 9, 10 gekoppelt. In jedem Verbinder 9, 10 ist ein U-förmig konfigurierter Ausgleichsabschnitt 11 bzw. 12 integriert. Diese weisen über das gesamte Stützgerüst alle in dieselbe Umfangsrichtung U.

Man erkennt, dass die Verbinder 9 bzw. 10 unterschiedlich lang ausgeführt sind. Die langen Verbinder 10 weisen beidseitig der Ausgleichsabschnitte 12 bogenförmige Schenkel 13 auf, die im Ausgangszustand A in dieselbe Umfangsrichtung U wie die Streben 5, 6 gekrümmt sind. Sowohl in Umfangsrichtung U als auch in Stentlängsachse L sind jeweils abwechselnd kurze Verbinder 9 und lange Verbinder 10 vorgesehen.

Auch die Streben 5, 6 sind bogenförmig konfiguriert, wobei alle Streben 5, 6 in dieselbe Umfangsrichtung U gekrümmt sind. Die Anschlüsse 14, 15 der in Stentlängsachse L aufeinander folgenden Verbinder 9 bzw. 10 an die Bogenabschnitte 4 liegen gegeneinander frontal gegenüber. Hierdurch ergibt sich ein vorteilhafter Kraftverlauf bzw. eine Krafteinleitung von den Knotenpunkten 16 zwischen den Streben 5, 6 bzw. den Ringsegmenten 3 und den Verbindern 9, 10.

Die Konfiguration des Stützgerüstes 2 bewirkt, dass im Stützzustand S des Stents 1 ein sich selbst stabilisierender Fachwerksverbund entsteht, wie dies in der Figur 3 durch Überlagerung eines theoretischen Modells auf das Stützgerüst 2 im Stützzustand S dargestellt ist. In dicker Linienführung ist der idealisierte angestrebte Fachwerkverbund dargestellt. Die in sich unverschieblichen Streben 5, 6 laufen jeweils in den Knotenpunkten 16 zusammen, wobei die Knotenpunkte 16 gelenkartig wirken. Diese Konfiguration stellt eine hohe Stabilität und Radialfestigkeit im Stützzustand S sicher. Im Ausgangszustand A kann der Stent 1 sehr gut auf kleine Durchmesser gekrümmt werden. Er lässt sich dann flexibel und sicher mit Hilfe eines Implantationsinstruments in der Regel eines Ballonkatheters in ein Körpergefäß in dem Bereich einer Stenose implantieren. Dort wird der Stent 1 expandiert. Dies kann selbsttätig erfolgen oder mit Hilfe eines Implantationsinstrumentes.

### Bezugszeichenaufstellung

- 1 -: Stent
- 2 -: Stützgerüst
- 3 -: Ringsegmente
- 4 -: Bogenabschnitte
- 5 -: Strebe
- 6 -: Strebe
- 7 -: Wellenberg
- 8 -: Wellental
- 9 -: Verbinder
- 10 -: Verbinder
- 11 -: Ausgleichsabschnitt
- 12 -: Ausgleichsabschnitt
- 13 -: Schenkel
- 14 -: Anschlüsse
- 15 -: Anschlüsse
- 16 -: Knotenpunkt

- A -: Ausgangszustand
- S -: Stützzustand
- L -: Stentlängsachse
- U -: Umfangsrichtung

## Patentansprüche

1. Stent mit einem tubulären Stützgerüst (2), welches von einem Ausgangszustand (A) in einen Stützzustand (S) aufweitbar ist und aus in Stentlängsachse (L) aufeinander folgenden Ringsegmenten (3) besteht, die aus sich in Umfangsrichtung (U) des Stützgerüstes (2) wellenförmig endlos aneinander schließenden Streben (5, 6) gebildet sind, wobei benachbarte Ringsegmente (3) durch unterschiedlich lange Verbinder (9, 10) mit U-förmig konfigurierten Ausgleichsabschnitten (11, 12) gekoppelt sind, **dadurch gekennzeichnet, dass** die Streben (5, 6) bogenförmig gekrümmt sind und über Bogenabschnitte (4) ineinander übergehen, wobei alle Streben (5, 6) in dieselbe Umfangsrichtung (U) gekrümmt sind und alle U-förmig konfigurierten Ausgleichsabschnitte (11, 12) der Verbinder (9, 10) in dieselbe Umfangsrichtung (U) weisen, wobei sowohl in Umfangsrichtung (U) als auch in Stentlängsachse (L) jeweils abwechselnd unterschiedlich lange Verbinder (9, 10) vorgesehen sind, und die langen Verbinder (10) beidseitig der Ausgleichsabschnitte (12) bogenförmige Schenkel (13) aufweisen, die in dieselbe Umfangsrichtung (U) wie die Streben (5, 6) gekrümmt sind.

2. Stent nach Anspruch 1, **dadurch gekennzeichnet, dass** einerseits die Wellenberge (7) und andererseits die Wellentäler (8) benachbarter Ringsegmente (3) einander frontal gegenüber liegen.

3. Stent nach einem der Ansprüche 1 oder 2, **dadurch gekennzeichnet, dass** die Anschlüsse (14, 15) der in Stentlängsachse (L) aufeinander folgenden Verbinder (9, 10) an die Bogenabschnitte (4) einander frontal gegenüber liegen.

## Claims

1. Stent with a tubular supporting frame (2), which can be expanded from an initial state (A) into a supporting state (S) and consists of annular segments (3) sequentially disposed in the longitudinal axis of the stent (L), said annular segments being formed by struts (5, 6) connected to each other continuously in a wave-like fashion in the peripheral direction (U) of the supporting frame (2), adjacent annular segments (3) being coupled by connectors (9, 10) of various lengths to U-shaped compensating sections (11, 12), **characterised in that** the struts (5, 6) are curved in the form of an arc and merge into each other via curved sections (4), with all struts (5, 6) being curved in the same peripheral direction (U) and all U-shaped compensating sections (11, 12) of the connectors (9, 10) pointing in the same peripheral direction (U), while both in the peripheral direction (U) and in the longitudinal axis of the stent (L), connectors (9, 10) having varying lengths are provided in an alternating manner, and the long connectors (10) have curved arms (13) on both sides of the compensating sections (12), said arms being curved in the same peripheral direction (U) as the struts (5, 6).

2. Stent according to claim 1, **characterised in that** on the one hand the wave crests (7) and on the other hand the wave troughs (8) of adjacent annular segments (3) lie frontally opposite each other.

3. Stent according to one of claims 1 or 2, **characterised in that** the connection points (14, 15) of the connectors (9, 10) sequentially disposed in the longitudinal axis of the stent (L) abut the curved sections (4) opposite each other.

## Revendications

1. Stent présentant une structure d'appui tubulaire (2) qui peut s'élargir d'un état initial (A) à un état d'appui (S) et est constitué de segments circulaires (3) se suivant consécutivement dans l'axe longitudinal du stent (L), qui sont formés de tiges (5, 6) adjointes de façon continue, de forme ondulée dans la direction périphérique (U) de la structure d'appui (2), des segments circulaires voisins (3) étant couplés par des éléments de liaison (9, 10) de longueurs différentes présentant des segments d'ajustement (11, 12) configurés en forme de U, **caractérisé en ce que** les tiges (5, 6) sont courbées en arc et passent les unes dans les autres par le biais de segments arqués (4), toutes les tiges (5, 6) étant courbées dans la même direction périphérique et tous les segments d'ajustement (11, 12) configurés en forme de U se tournant vers les éléments de liaison (9, 10) dans la même direction périphérique (U), des éléments de liaison (9, 10) différents respectivement et alternativement, à la fois dans la direction périphérique (U) et également dans l'axe longitudinal du stent (L) étant prévus, et les long éléments de liaison (10) présentant des deux côtés des segments d'ajustement (12) des branches arquées (13) qui sont courbées dans la même direction périphérique (U) que les tiges (5, 6).

2. Stent selon la revendication 1, **caractérisé en ce que** d'une part les sommets des vagues (7) et d'autre part, les creux des vagues (8) de segments circulaires voisins (3) sont opposés frontalement les uns aux autres.

3. Stent selon l'une des revendications 1 ou 2, **caractérisé en ce que** les raccords (14, 15) des éléments de liaison se suivant consécutivement dans l'axe longitudinal du stent (L) sont opposés frontalement les uns aux autres au segment arqué (4).
